# EUROPEAN PATENT SPECIFICATION

(11) **EP 1 762 214 B1**
(45) Date of publication and mention of the grant of the patent: **31.03.2010**
(21) Application number: 05108394.7
(22) Date of filing: 13.09.2005
(51) Int. Cl.: A61K 6/10

(54) **Method of dental impression taking**
Verfahren zum Abdrucknehmen
Procédé pour la prise d'empreintes

(43) Date of publication of application: 14.03.2007
(73) Proprietor: Coltène AG, 9450 Altstätten (SG) (CH)
(72) Inventor: Lampl, Stephan, 9450, Luechingen (CH); Dr.Luebbers, Dierk, 9453, Eichberg (CH); Dr. Kollefrath Ralf, 9464 Rüti (DE)
(74) Representative: Hepp, Dieter

(56) References cited:
- EP-A- 0 522 341
- EP-A- 1 498 099
- WO-A-96/15179
- US-A- 5 975 906
- VILCHES JORGE JUAN; NAVARRO XAVIER: "New silicones for the evaluation of sudomotor function with the impression mold technique" CLINICAL AUTONOMIC RESEARCH : OFFICIAL JOURNAL OF THE CLINICAL AUTONOMIC RESEARCH SOCIETY, vol. 12, no. 1, February 2002 (2002-02), pages 20-23, XP002378984
- "Using PolySil TransBite VPS material to create a custom matrix by SciCan" DENTAL PRODUCTS REPORT, vol. 30, 1996, pages 62-63, XP008060062
- RAWICZ ANDREW H ; MELNYK IVAN ; KOWALSKI PAWEL: "Translucency measurements in teeth and dental materials" PROC SPIE INT SOC OPT ENG; PROCEEDINGS OF SPIE - THE INTERNATIONAL SOCIETY FOR OPTICAL ENGINEERING; LASERS IN DENTISTRY IX; SAN JOSE, CA, UNITED STATES, vol. 4950, 2003, pages 259-265, XP002378968

## Description

The present invention relates to the technical field of dental impression taking and a polymeric material which has been found especially useful therefore.

In the course of various dental applications, e.g. in dental restoration, it is necessary to take an impression of the patient's dental situation, in order to provide the dentist with a 3D model. Such a 3D model needs to resemble the dental situation as exactly as possible in order to provide the dentist with a suitable basis e.g. for a satisfying restoration being prepared, such as a crown, a bridge, or the like.

A common problem in dental impression taking is a loss in precision of the mold due to irregularities, e.g. inclusion of air bubbles within or under the impression material, resulting in non-molded areas, which are not reproduced. Such non-molded areas are afterwards either extrapolated by the dentist, if possible (often resulting in non-satisfactory results) or the impression taking must be performed once again in the worst case, causing inconvenience to both the patient and the dentist. In the state of the art, inclusion of air bubbles within or under the impression material was tried to be eliminated by various approaches, e.g. by modifying the flow-characteristics of the impression material, or by using special impression trays, which aims to eliminate such air inclusions by applying reduced pressure to the impression region. However, all these approaches do not allow for a reliable elimination of said air inclusions under all circumstances, and/or afford complicated impression tray devices.

Transparent dental impression materials as such are disclosed in US 5,975,906 (Knutson), WO 96/15179 (Waller), EP 0 522 341 Al (Voigt et al.) and Dental Products Report, vol. 30 (1996), pages 62-63 ("Using Polysil TransBite VPS material to create a custom matrix by SciCan").

A comparison of various commercially available, transparent dental impression materials can be found in Vilches et al., Clinical Autonomic Research, vol. 12 (2002), pages 20-23 ("New silicones for the evaluation of sudomotor function with the impression mold technique").

It was thus an object of the present invention to overcome the drawbacks of the prior art, especially to more reliably allow for the elimination of irregularities, e.g. the inclusion of air bubbles within and/or under an impression material, in order to more reliably allow for a highly detailed impression taking.

This object has been solved by a method of dental impression taking and a polymeric material therefore, as outlined below.

According to the invention, a method of dental impression taking comprises the step of applying a polymeric material at least partially to the region to be reproduced by the impression, wherein the polymeric material is translucent. Preferably, the translucency of the polymeric material is in the range of about 30% to about 100%, preferably of ≥ 40%, most preferably of ≥ 50%.

Preferably, the method of impression taking comprises the additional steps of:
- identifying an irregularity, especially air inclusion(s) within and/or under said translucent polymeric material;
- eliminating said irregularities before hardening of said translucent polymeric material.

To the best of the applicant's knowledge, the use of a polymeric, translucent material comprising finely dispersed fillers in dental impression taking has never been suggested, not to mention been ever practiced. In contrast, commonly used impression materials (silicon materials are mostly used) are opaque, . However, according to the invention, the above-mentioned drawbacks of the prior art can surprisingly be overcome by simply using a translucent impression material comprising finely dispersed fillers, because a sufficient translucency allows for easy identification of possible air inclusion(s). Such identified air inclusion(s) can then easily be eliminated before fied air inclusion(s) can then easily be eliminated before hardening of the applied impression material, e.g. by simply sticking and/or slightly turning the application tip of common dispensers of impression material at the said air inclusion(s), whereupon the air inclusion(s) can be easily eliminated.

Preferably, the process of dental impression taking according to the invention is a process chosen from the group consisting of two-material-two-phase processes, two-material-one-phase processes, and one-material-one-phase processes. These techniques are perfectly known by the person of routine skill in the art.

According to the two-material-two-phase process ("Knetmassenoder Korrekturabformtechnik"), a crude impression is taken with a kneadable or heavy body impression material, which is subsequently additionally manipulated e.g. with cutting instruments outside the patient's mouth, and finally a correction impression material is applied onto said manipulated crude impression again into the patient's mouth to a final impression mold. If put into practice according to the invention, a translucent impression material is to be used at least as the correction impression material; if wanted and found appropriated in a special case, both impression materials may be translucent.

According to the two-material-one-phase process ("Doppelmischtechnik"), two impression materials are applied at the same time, the one afar from the tooth and the other adjacent to the tooth. If put into practice according to the invention, a translucent impression material is to be used at least adjacent to the tooth; if wanted and found appropriated in a special case, both impression materials may be translucent.

According to the one-material-one-phase process ("Monophasenabformtechnik"), one and the same material is applied in an impression tray and additionally from besides, e.g. with a syringe. If put into practice according to the invention, a translucent impression material is to be used.

The invention moreover relates to a polymeric material for dental applications, especially for impression taking, characterized in that it exhibits the following features:
- comprising finely dispersed fillers
- a translucency in the range of about 30% to about 100%, preferably of ≥ 40%, most preferably of ≥ 50%;
- a shore A hardness according to DIN 53505 (Ausgabe 08/1973) in the range of about 20 to about 70, preferably of about 30 to about 60, most preferably of about 45 to 55;
- a tensile strength according to DIN 53504 (Ausgabe 05/1969) in the range of about 0.2 MPa to about 7 MPa, preferably of about 1 MPa to about 6 MPa, most preferably of about 1.5 MPa to about 5.5 MPa.

The translucency in the above-mentioned ranges has proven sufficient, the high translucencies ideal for visual control of the applied material for the presence of air bubbles. The sample is prepared by filling the uncured material in a stainless steel form of 25*20*1 mm, and pressing off excessive material with a glass plate. After curing at 23°C, the sample is taken out translucency of the polymeric material is determined through the 1 mm dimension of the sample with a BaSO₄ white background in a US/VIS spectrophotometer (LAMBDA 16, Perkin Elmer) with "UL-BRICHTscher Kugel". The background correction is measured against a BaSO₄ white standard.

The above-mentioned ranges of shore A hardness according to DIN 53505 and tensile strength according to DIN 53504 have proven excellent for putting into practice the materials according to all embodiments of the present invention. The person of routine skill in the art can easily choose and adapt the shore A hardness and the tensile strength in the above-mentioned ranges, by routine laboratory techniques, e.g. incorporation of suitable additives which on the other hand must not hamper the above-mentioned translucency. If e.g. fillers are wanted to be incorporated, they must be chosen suchlike (i.e. suitably fine dispersed) and in such an amount, that the translucency requirement is fulfilled.

According to yet another preferred embodiment, the material exhibits a consistency according to DIN ISO 4823:2000 of type 1 to type 3, preferably of type 2 or type 3.

Currently, hydrophilic impression materials are preferred in the art. Accordingly, the material according to the invention preferably exhibits a wetting angle of contact of less than about 50° after 2 minutes. However, the invention is not limited to hydrophilic materials, i.e. angles of contact of more than about 50° after 2 minutes, especially of more than about 90° after 2 minutes may be found also appropriate for some applications. The angle of contact is determined as follows: a polymeric sample is prepared in a brass frame of 65*25*3 mm size and cured therein for about 10 minutes. Five minutes after detaching the sample from the frame, a droplet of deionized water is dropped onto the sample surface, and the angle of contact is determined with a drop shape analysis system DSA 10 of KRÜSS GmbH, Hamburg, Germany. If so desired, the person of routine skill in the art will easily achieve and/or finetune a suitable angle of contact e.g. by incorporating surfactants of common practice in the art, e.g. branched-nonylphenol ethoxylate (IGEPAL BC4), etc.

According to an especially preferred embodiment of the present invention, the polymeric material is a silicon-based material, preferably an addition-crosslinked silicon material. Also condensation-crosslinked silicon materials are however appropriate.

As used here and henceforth, the term "addition-crosslinked" or "addition-crosslinkable" means that the polymer comprises at least one functional group which may react with a crosslinking agent via an addition reaction. A typical example is that the polymer comprises at least one vinyl group, preferably two vinyl groups, which may undergo an electrophilic addition reaction with an appropriate crosslinking agent. Preferably, these vinyl groups are terminal.

As used here and henceforth, the term "condensation-crosslinked" or "condensation-crosslinkable" means that the polymer comprises at least one functional group which may react with a crosslinking agent via a condensation reaction. A typical example is that the polymer comprises at least one hydroxyl group, preferably two hydroxyl groups, which may undergo a condensation reaction with an appropriate crosslinking agent, for example a crosslinking agent comprising alkoxy silicates.

More precisely, the polymeric material is or comprises a polyorganosiloxane, comprising building blocks suitably chosen from (but not necessarily comprising all of them) [M] (R₃SiO_{1/2}), [D] (R₂SiO_{2/2}), [T] (RSiO_{3/2}) and Q (SiO_{4/2}). The polyorganosiloxane may be linear, branched, cyclic and/or preferably crosslinked. The polyorganosiloxane is preferably modified by hydrosilylation, i.e. the addition of silanes and/or (poly) (organo)siloxanes comprising Si,H bonds to unsaturated groups, e.g. the vinyl groups of the above-mentioned polyorganosiloxanes. The person of routine skill in the art will readily choose a suitable composition of a polyorganosiloxane in order to meet the above-defined functional requirements of the polymeric material.

Alternatively, the polymeric material may also be or comprise a polyether-based material, especially preferred an aziridine-crosslinked polyether material. It is evident to the person of routine skill in the art, how to adjust the functional requirements as outlined above in the context of silicon-based material also for a polyether-based material.

Moreover, the molecular weight of the polymeric material is chosen such that the required shore A hardness and tensile strength are obtained. Optionally, additives such as rheology modifiers may be added for adjustment of the said parameters, as it is routine skill in the art. Especially dyes or pigments (such as e.g. fluorescent pigments, e.g. Lumilux Blau LZ (Omya AG), or glimmer pigments such as Timica Extra Bright 1500 (Mimox (LCW))) may be added in suitable amounts to the composition, as long as the translucency and the other critical parameters as outlined above are not hampered. Moreover, fillers are added to the composition as long as the translucency and the other critical parameters as outlined above are not hampered, e.g. finely dispersed fillers, preferably nanofillers (e.g. Aerosil), and/or fillers which inherently exhibit a suitable refractive index (e.g. MgF₂). The viscosity of the uncured material is suitably adjusted in the range of about 0.5 to about 500 Pa*s, preferably about 10 to about 400 Pa*s, more preferably about 100 to about 300 Pa*s, as measured according to Brookfield. In any case, the rheology of the mixture to be applied is adjusted suchlike to allow for application by conventional dispensers, e.g. manually operated double chamber cartridges.

Yet another aspect of the present invention relates to a kit of parts, comprising a translucent polymeric material comprising fillers preferably as outlined above, and a further polymeric, preferably translucent material. Such a kit of parts can be manufactured and shipped as it is current state of the art with all one-phase and two-phase processes in dental impression taking as outlined in the introductory part. According to the invention, however, at least one, if desired both polymeric materials are translucent.

According to another aspect of the present invention, the material according to the invention is used for the preparation of a dental impression chosen from the group consisting of (i) a key for temporary or definitive composite crowns, telescope crowns or bridges; (ii) a key for composite facings or veneers; (iii) a positioning key for orthodontic brackets; (iv) a positioning key prior to insertion of a dental implant; (v) an implant template matrix; (vi) a (pre)impression for build-up of anterior and posterior teeth in restorative dentistry.

All the above-mentioned applications may exploit the same inherent advantages of a translucent material: Firstly, irregularities, bubbles or the like can be easily identified and eliminated before hardening. Secondly, the translucency of the material also allows for light-hardening of a suitably chosen, light-curing further material subsequently filled into the impression (e.g. to prepare a final model) by simply irradiating through the translucent material.

The invention will now be explained in more detail by a detailed description of preferred embodiments;

### 1. Compositions

The following two-component polymeric silicon materials were prepared:

### A. "Light Body" (SiH/Vinyl: 1.94)

| Base Paste: | | |
|---|---|---|
| 70.00 g | Silopren Grundmischung P300 from GE Bayer | (0.05 mmol Vinyl/g) |
| 9.00 g | Silopren Crosslinker 4.3 from GE Bayer | (4.20 mmol SiH/g) |
| 2.00 g | Silopren Chain Extender TP 3359 from GE Bayer | (1.42 mmol SiH/g) |
| 9.00 g | Vinylsilicon VS 50 from Hanse Chemie, an alpha/omega-Divinylpolydimethylsiloxan with 50 mPa*s | (0.63 mmol Vinyl/g) |
| 10.00 g | Vinylsilicon VS 10,000 from Hanse Chemie, an alpha/omega-Divinylpolydimethylsiloxan with 10,000 mPa*s | (0.05 mmol Vinyl/g) |

| Catalyst Paste: | | |
|---|---|---|
| 70.00 g | Silopren Grundmischung P300 from GE Bayer | (0.05 mmol Vinyl/g) |
| 0.35 g | Catalyst preparation (90 weight% alpha/omega-divinylpolydimethylsiloxan with 1,000 mPa*s; 10 weight% catalyst complex "Karstedt"; corr. to 4 weight% pure Pt) | (0.53 mmol Vinyl/g) |
| 10.00 g | Vinylsilicon VS 50 from Hanse Chemie, an alpha/omega-Divinylpolydimethylsiloxan with 50 mPa*s | (0.63 mmol Vinyl/g) |
| 0.025 g | Inhibitor PTS-I 27 (DVTMDS) from Wacker Chemie | (10.75 mmol Vinyl/g) |
| 19.70 g | Vinylsilicon VS 10,000 from Hanse Chemie, an alpha/omega-Divinylpolydimethylsiloxan with 10,000 mPa*s | (0.05 mmol Vinyl/g) |

### B. "Regular Body" (SiH/Vinyl: 1.87)

| Base Paste: | | |
|---|---|---|
| 40.00 g | Silopren Grundmischung P1,300 from GE Bayer | (0.06 mmol Vinyl/g) |
| 9.00 g | Silopren Crosslinker 4.3 from GE Bayer | (4.20 mmol SiH/g) |
| 2.00 g | Silopren Chain Extender TP 3359 from GE Bayer | (1.42 mmol SiH/g) |
| 9.00 g | Vinylsilicon VS 50 from Hanse Chemie, an alpha/omega-Divinylpolydimethylsiloxan with 50 mPa*s | (0.63 mmol Vinyl/g) |
| 10.00 g | Vinylsilicon VS 10,000 from Hanse Chemie, an alpha/omega-Divinylpolydimethylsiloxan with 10,000 mPa*s | (0.05 mmol Vinyl/g) |
| 30.00 g | Silopren Grundmischung P300 from GE Bayer | (0.05 mmol Vinyl/g) |
| Catalyst Paste: | | |
| 40.00 g | Silopren Grundmischung P1,300 from GE Bayer | (0.06 mmol Vinyl/g) |
| 0.30 g | Catalyst preparation (90 weight% alpha/omega-divinylpolydimethylsiloxan with 1,000 mPa*s; 10 weight% catalyst complex "Karstedt"; corr. to 4 weight% pure Pt) | (0.53 mmol Vinyl/g) |
| 10.00 g | Vinylsilicon VS 50 from Hanse Chemie, an alpha/omega-Divinylpolydimethylsiloxan with 50 mPa*s | (0.63 mmol Vinyl/g) |
| 0.025 g | Inhibitor PTS-I 27 (DVTMDS) from Wacker Chemie | (10.75 mmol Vinyl/g) |
| 19.70 g | Vinylsilicon VS 10,000 from Hanse Chemie, an alpha/omega-Divinylpolydimethylsiloxan with 10,000 mPa*s | (0.05 mmol Vinyl/g) |
| 30.00 g | Silopren Grundmischung P300 from GE Bayer | (0.05 mmol Vinyl/g) |

### C. "Regular Body & Tensid" (SiH/Vinyl: 1.87)

| Base Paste: | | |
|---|---|---|
| 39.00 g | Silopren Grundmischung P1,300 from GE Bayer | (0.06 mmol Vinyl/g) |
| 9.00 g | Silopren Crosslinker 4.3 from GE Bayer | (4.20 mmol SiH/g) |
| 2.00 g | Silopren Chain Extender TP 3359 from GE Bayer | (1.42 mmol SiH/g) |
| 9.00 g | Vinylsilicon VS 50 from Hanse Chemie, an alpha/omega-Divinylpolydimethylsiloxan with 50 mPa*s | (0.63 mmol Vinyl/g) |
| 10.00 g | Vinylsilicon VS 10,000 from Hanse Chemie, an alpha/omega-Divinylpolydimethylsiloxan with 10,000 mPa*s | (0.05 mmol Vinyl/g) |
| 30.00 g | Silopren Grundmischung P300 from GE Bayer | (0.05 mmol Vinyl/g) |
| 1.00 g | IGEPAL BC 4 (Rhodia) | |
| 40.00 g | Silopren Grundmischung P1,300 from GE Bayer | (0.06 mmol Vinyl/g) |
| Catalyst paste: | | |
| 0.30 g | Catalyst preparation (90 weight% alpha/omega-divinylpolydimethylsiloxan with 1,000 mPa*s; 10 weight% catalyst complex "Karstedt"; corr. to 4 weight% pure Pt) | (0.53 mmol Vinyl/g) |
| 10.00 g | Vinylsilicon VS 50 from Hanse Chemie, an alpha/omega-Divinylpolydimethylsiloxan with 50 mPa*s | (0.63 mmol Vinyl/g) |
| 0.025 g | Inhibitor PTS-I 27 (DVTMDS) from Wacker Chemie | (10.75 mmol Vinyl/g) |
| 19.70 g | Vinylsilicon VS 10,000 from Hanse Chemie, an alpha/omega-Divinylpolydimethylsiloxan with 10,000 mPa*s | (0.05 mmol Vinyl/g) |
| 30.00 g | Silopren Grundmischung P300 from GE Bayer | (0.05 mmol Vinyl/g) |

### 2. Measurement results

Base paste and catalyst paste of all above-mentioned compositions were homogeneously mixed in equal amounts (50:50), cured and typical features of the resulting masses were determined as follows (if not mentioned otherwise, the methods as outlined herein above were applied for the measurements):

| Comp. | consistency | Tear strength | Wetting | Shore | Translucency |
|---|---|---|---|---|---|
| | | [MPa] | angle | A | [%] |
| A | 43 mm | 2.92 | > 90° | 44 | 67.6 |
| | (type 3) | | | | |
| B | 39 mm | 4.76 | > 90° | 43 | 67.4 |
| | (type 2) | | | | |
| C | 35 | 4.22 | 45° | 43 | 63.3 |
| | (type 2) | | | | |

All compositions A, B and C proved highly suitable in dental impression taking, as outlined above.

## Claims

1. A method of dental impression taking, comprising the step of applying a polymeric material at least partially to the region to be reproduced by the impression, wherein the polymeric material is translucent,
**characterized in that** the polymeric material
- comprises finely dispersed fillers; and
- exhibits a consistency according to DIN ISO 4823:2000 of type 2 or 3, and comprising the additional steps of:
- identifying an irregularity, especially air inclusion(s) within and/or under said translucent polymeric material;
- eliminating said irregularities before hardening of said translucent polymeric material.

2. A method according to claim 1,
**characterized in that** the polymeric material exhibits a translucency in the range of 30% to 100%.

3. A method according to one of the preceding claims, **characterized in that** the process of dental impression taking is a process chosen from the group consisting of two-material-two-phase processes, two-material-one-phase processes, and one-material-one-phase processes.

4. A Silicon-based polymeric material for dental applications, **characterized in that** it exhibits the following features:
- a translucency in the range of 30% to 100%;
- a shore A hardness according to DIN 53505 in the range of 20 to 70;
- a tensile strength according to DIN 53504 in the range of 0.2 MPa to 7 MPa,
wherein the polymeric material
- comprises finely dispersed fillers; and
- exhibits a consistency according to DIN ISO 4823:2000 of type 2 or 3.

5. A material according to claim 4, **characterized in that** it exhibits a wetting angle of contact of less than 50° after 2 minutes.

6. A kit of parts, comprising a translucent polymeric material according to one of claims 4 or 5, and a further polymeric material which is non-translucent or translucent.

7. A dental impression which is at least partially translucent, obtainable from a material according to one of claims 4 to 5 and/or by method according to one of claims 1 to 3.

8. Use of a material according to one of claims 4 to 6 for preparation of a dental impression chosen from the group consisting of (i) a key for temporary or definitive composite crowns, telescope crowns or bridges; (ii) a key for composite facings or veneers; (iii) a positioning key for orthodontic brackets; (iv) a positioning key prior to insertion of a dental implant; (v) an implant template matrix; (vi) a (pre)impression for build-up of anterior and posterior teeth in restorative dentistry.

## Patentansprüche

1. Verfahren zur Abnahme einer Zahnabformung, bei dem in einem schritt ein Polymermaterial zumindest teilweise auf den Bereich angewendet wird, der durch die Abformung dargestellt werden soll, wobei das Polymermaterial lichtdurchlässig ist, **dadurch gekennzeichnet, dass** das Polymermaterial
- fein dispergierte Füllstoffe umfasst und
- eine Konsistenz gemäß DIN ISO 4823:2000 des Typs 2 oder 3 aufweist,
und bei dem in weiteren Schritten:
- eine Unregelmäßigkeit, insbesondere ein Lufteinschluss oder Lufteinschlüsse, in und/oder unter dem lichtdurchlässigen Polymermaterial identifiziert wird;
- die Unregelmäßigkeiten vor dem Härten des lichtdurchlässigen Polymermaterials beseitigt werden.

2. Verfahren nach Anspruch 1, **dadurch gekennzeichnet, dass** das Polymermaterial eine Lichtdurchlässigkeit im Bereich von 30% bis 100% aufweist.

3. Verfahren nach einem der vorhergehenden Ansprüche, **dadurch gekennzeichnet, dass** das Verfahren zur Abnahme von Zahnabformungen ein Verfahren ist, das ausgewählt ist aus der Gruppe, bestehend aus Zwei-Materialien-Zweiphasen-Verfahren, Zwei-Materialien-Einphasen-Verfahren und Ein-Material-Einphasen-Verfahren.

4. Polymermaterial auf silikonbasis für Zahnanwendungen, **dadurch gekennzeichnet, dass** es folgende Merkmale aufweist:
- eine Lichtdurchlässigkeit im Bereich von 30% bis 100%;
- eine Shore-A-Härte gemäß DIN 53505 im Bereich von 20 bis 70;
- eine Zugfestigkeit gemäß DIN 53504 im Bereich von 0,2 MPa bis 7 MPa;
wobei das Polymermaterial
- fein dispergierte Füllstoffe umfasst und
- eine Konsistenz gemäß DIN ISO 4823:2000 des Typs 2 oder 3 aufweist.

5. Material nach Anspruch 4, **dadurch gekennzeichnet, dass** es einen Benetzungskontaktwinkel von weniger als 50° nach 2 Minuten aufweist.

6. Set, umfassend ein lichtdurchlässiges Polymermaterial nach einem der Ansprüche 4 oder 5 und ein weiteres Polymermaterial, das nicht lichtdurchlässig oder lichtdurchlässig ist.

7. Zahnabformung, die zumindest teilweise lichtdurchlässig ist und aus einem Material nach einem der Ansprüche 4 bis 5 und/oder durch ein Verfahren nach einem der Ansprüche 1 bis 3 erhältlich ist.

8. Verwendung eines Materials nach einem der Ansprüche 4 bis 6 zur Herstellung einer Zahnabformung, ausgewählt aus der Gruppe, bestehend aus (i) einer Passform für temporäre oder definitive Verbund-Zahnkronen, Teleskopkronen oder -brücken; (ii) einer Passform für Verbund-Facetten oder -Veneers; (iii) einer Positionierpassform für orthodontische Zahnspangen; (iv) eine Positionierpassform vor dem Einsetzen eines Zahnimplantats; (v) einer Implantatmatrizenmatrix; (vi) einer (Vor-) Abformung zum Aufbauen von anterioren oder posterioren Zähnen bei der restaurativen Zahnheilkunde.

## Revendications

1. Méthode de prise d'empreinte dentaire, comprenant l'étape consistant à appliquer un matériau polymère au moins partiellement à la région à reproduire par empreinte, dans laquelle le matériau polymère est translucide, **caractérisée en ce que** le matériau polymère
- comprend des agents de remplissage finement dispersés ; et
- présente une consistance de type 2 ou 3 conformément à la norme DIN ISO 4823:2000, et
comprenant en outre les étapes consistant à:
- identifier une irrégularité, en particulier une ou des inclusion(s) d'air dans et/ou sous ledit matériau polymère translucide;
- éliminer lesdites irrégularités avant de procéder au durcissement dudit matériau polymère translucide.

2. Méthode selon la revendication 1, **caractérisée en ce que** le matériau polymère présente une translucidité allant de 30% à 100%.

3. Méthode selon l'une des revendications précédentes, **caractérisée en ce que** le procédé de prise d'empreinte dentaire est un procédé choisi dans le groupe consistant en les procédés à base de deux matériaux formant deux phases, les procédés à base de deux matériaux formant une phase, et les procédés à base d'un matériau formant une phase.

4. Matériau polymère à base de silicium destiné à des applications dentaires, **caractérisé en ce qu'**il présente les caractéristiques suivantes:
- une translucidité allant de 30% à 100%;
- une dureté shore A conformément à la norme DIN 53505 allant de 20 à 70;
- une résistance à la traction conformément à la norme DIN 53504 allant de 0,2 MPa à 7 MPa,
le matériau polymère
- comprenant des agents de remplissage finement dispersés; et
- présentant une consistance de type 2 ou 3 conformément à la norme DIN ISO 4823:2000.

5. Matériau selon la revendication 4, **caractérisé en ce qu'**il présente un angle de mouillage de contact inférieur à 50° après 2 minutes.

6. Kit comprenant un matériau polymère translucide selon l'une des revendications 4 ou 5, et un autre matériau polymère qui est non translucide ou translucide.

7. Empreinte dentaire qui est au moins partiellement translucide, qui peut être obtenue à partir d'un matériau selon l'une des revendications 4 ou 5 et/ou par la méthode selon l'une des revendications 1 à 3.

8. Utilisation d'un matériau selon l'une des revendications 4 à 6 pour la préparation d'une empreinte dentaire choisie dans le groupe consistant en (i) un moule pour des couronnes composites provisoires ou définitives, des couronnes télescopiques ou des bridges; (ii) un moule pour des revêtements ou des facettes composites; (iii) un moule de positionnement pour des appareils orthodontiques; (iv) un moule de positionnement avant l'insertion d'un implant dentaire; (v) une matrice amovible d'implant; (vi) une (pré)empreinte pour la reconstitution des dents antérieures et postérieures en dentisterie restauratrice.
